# EUROPEAN PATENT APPLICATION

(11) **EP 2 273 272 A1**
(43) Date of publication of application: **12.01.2011**
(21) Application number: 09164615.8
(22) Date of filing: 06.07.2009
(51) Int. Cl.: G01N 33/574

(54) **Method for predicting the outcome of chemotherapy in ovarian cancer**

(71) Applicant: Stichting Katholieke Universiteit, 6500 HB Nijmegen (NL)
(72) Inventor: Massuger, Leonardus Franciscus Adrianus Gerardus, 6500 HB Nijmegen (NL); Wevers, Ronald Allan, 6681 EZ Bemmel (NL)
(74) Representative: Habets, Winand

(57) **Abstract**

The invention is in the field of molecular medicine and provides methods for predicting the outcome of chemotherapy in ovarian cancer. It also provides methods for the screening of test compounds that may improve the efficacy of a chemotherapeutic agent in the chemotherapy of ovarian cancer. The invention provides an in vitro method for predicting the outcome of chemotherapy in ovarian cancer comprising the steps of providing a test sample comprising ovarian cyst fluid, determining the level of alpha-1 acid glycoprotein in the test sample and comparing the level of alpha-1 acid glycoprotein in the test sample with a control level of alpha-1 acid glycoprotein wherein a level of alpha-1 acid glycoprotein in the test sample higher than a control level of alpha-1 acid glycoprotein indicates that the individual is at an increased risk for recurrent disease.

## Description

### Field of the invention

The invention is in the field of molecular medicine and provides methods for predicting the outcome of chemotherapy in ovarian cancer. It also provides methods for the screening of test compounds that may improve the efficacy of a chemotherapeutic agent in the chemotherapy of ovarian cancer.

### Background of the invention

Epithelial ovarian cancer (EOC) is the most lethal gynaecological malignancy world-wide. Due to a lack of specific clinical symptoms in an early stage of disease, approximately 70% of the patients is diagnosed with advanced stage of disease (FIGO III and IV) with 5 year survival rates of only 10-20% [1]. The major determinants of clinical outcome are represented by the extent of residual tumor after primary surgery, and sensitivity to platinum-based chemotherapy [2,3].

Biomarkers for ovarian cancer are known in the art. WO2006010047 describes methods and compositions for identifying ovarian cancer in a patient sample. The methods comprise detecting overexpression of at least one biomarker in a body sample, wherein the biomarker is selectively overexpressed in ovarian cancer. The biomarkers revealed therein are acute phase reactants, lipoproteins, proteins involved in the regulation of the complement system, regulators of apoptosis, proteins that bind hemoglobin, heme, or iron, cytostructural proteins, enzymes that detoxify metabolic byproducts, growth factors, and hormone transporters. Overexpression is detected therein at the protein level using biomarker-specific antibodies or at the nucleic acid level using nucleic acid hybridization techniques.

WO2006089212 discloses the discovery that urinary Cyr61 protein levels are upregulated in patients that have cancers of epithelial origin, i.e. breast cancer and ovarian cancer. It reveals that the amount of Cyr61 protein detected in a urine sample correlates with disease status such that Cyr61 levels can be used to predict the presence of, as well as the metastatic potential of cancer.

WO2008121340 is concerned with protein-based biomarkers and biomarker combinations that are useful in qualifying ovarian cancer status in a patient. In particular, the biomarkers of this invention can be detected by SELDI mass spectrometry. Exemplified markers are IL-8, IL-8 (6-77) and MCP.

WO2007002264 describes a method for qualifying ovarian cancer status in a subject comprising the steps of measuring at least one biomarker including a CTAP3 -related protein in a biological sample from the subject and correlating the measurement with ovarian cancer status.

WO2006099126 provides protein-based biomarkers and biomarker combinations that are useful in qualifying ovarian cancer status as well as endometrial cancer status in a patient. In particular, it is described that hepcidin is a biomarker for both ovarian cancer and endometrial cancer and that a panel of biomarkers, including hepcidin, transthyretin and optionally other markers are useful to classify a subject sample as ovarian cancer or non- ovarian cancer.

WO2007002527 describes a method for qualifying ovarian cancer status in a subject comprising the steps of measuring at least one biomarker in a biological sample from the subject, wherein the at least one biomarker is selected from the group consisting of platelet factor 4, beta-2-microglobulin, albumin, vitamin D binding protein and transferrin and (b) correlating the measurement with ovarian cancer status.

US2008038754 is concerned with a method of screening for ovarian neoplasia in a subject, comprising the steps of measuring hemoglobin in a tissue sample of the subject and comparing the measured hemoglobin of the tissue sample to a measurement of hemoglobin in normal tissue, wherein a two-fold or greater increase in the measured of hemoglobin of the tissue sample compared to the measurement of hemoglobin in normal tissue is indicative of ovarian neoplasia.

WO2007098102 describes a method for determining if an individual is at risk of developing, or has developed, cancer, comprising the steps of determining a level of free urinary neutrophil gelatinase associated lipocalin (NGAL) in a test urine sample obtained from an individual; comparing the level of free NGAL in the test urine sample with a control level of free NGAL; wherein a level of free NGAL in the test sample higher than a control level of free NGAL indicates that the individual is either at increased risk for developing cancer, or has cancer.

WO2007061656 discloses a method of detecting ovarian cancer in a female test subject comprising determining the amount of plasmenyl-PA or a biomarker having a mass charge ratio of approximately 655.3 in a sample of a bodily fluid taken from the female test subject and comparing the amount of plasmenyl-PA (or the biomarker) in the sample of the bodily fluid taken from the female test subject to a range of amounts of plasmenyl-PA (or the biomarker) found in samples of bodily fluids taken from a group of normal female subjects of the same species as the female test subject and lacking ovarian cancer, whereby a lower amount of the plasmenyl-PA (or the biomarker) in the sample of the bodily fluid taken from the female test subject indicates the presence of ovarian cancer.

WO2005098446 relates to a method of qualifying ovarian cancer status in a subject comprising the steps of measuring at least one biomarker in a sample from the subject, wherein the biomarker is selected from the group consisting of ApoAl, modified ApoAl, transthyretin AN 10, native transthyretin, cysteinylated transthyretin, sulfonated transthyretin, CysGly modified transthyretin, glutathionylated transthyretin, IAIH4 fragment no. 1, IAIH4 fragment no. 2, and IAIH4 fragment no. 3, and combinations thereof, and correlating the measurement with ovarian cancer status

US2006073525 discloses a method for detecting or screening a subject for breast or ovarian cancer comprising the steps of obtaining a biological sample from a subject, detecting the amount of kallikrein 5 in said sample; and comparing said amount of kallikrein 5 detected to a predetermined standard, where detection of a level of kallikrein 5 greater than that of a standard is indicative of breast or ovarian cancer.

FIGO stage has been the most important prognostic factor in ovarian cancer for several decades [22]. For this reason, patients with advanced FIGO stage receive chemotherapy treatment after primary surgery. However, additional tumor markers are needed to identify response to chemotherapy in order to individualize treatment, since 20% of the patients shows resistance to cisplatinum [3].

At present, CA 125 in serum is the only tumor marker to monitor the response to chemotherapy, recurrence and disease progression in patients with EOC. The role of preoperative serum CA 125 as a prognostic tumor marker remains unclear as most studies could not show a significantly independent prognostic effect for CA 125 since its values are highly correlated with FIGO stage [23]. In addition, CA 125 cannot be used as a marker for mucinous EOC because its expression is absent or reduced in these tumors [24].

None of the above described markers have as yet provided a sensitivity and specificity that completely satisfy the needs of clinicians. Therefore, a further clinical need exists for further ovarian tumor markers, in particular pre-treatment tumor markers predicting response to cisplatinum.

### Summary of the invention

We have found that alpha-1 acid glycoprotein (AGP) concentrations were significantly higher in cyst fluid of patients with ovarian cancer (n=53) as compared to AGP concentrations in cyst fluid of patients with benign (n=22) and borderline (n=11) ovarian tumors. We also found that AGP concentrations were significantly higher in cyst fluid of patients with a poor disease outcome compared to patients with a relatively good disease outcome. Moreover, AGP seemed to be an independent prognostic factor for survival in multivariate Cox regression analysis.

Accordingly, the invention provides an in vitro method for predicting the outcome of chemotherapy in ovarian cancer comprising the steps of providing a test sample comprising ovarian cyst fluid, determining the level of alpha-1 acid glycoprotein in the test sample and comparing the level of alpha-1 acid glycoprotein in the test sample with a control level of alpha-1 acid glycoprotein wherein a level of alpha-1 acid glycoprotein in the test sample higher than a control level of alpha-1 acid glycoprotein indicates that the individual is at an increased risk for recurrent disease.

We also found that high levels of AGP in cyst fluid of these ovarian cancer patients lead to an increased metabolism of chemotherapeutics and thus to a diminished effect of the chemotherapy on the tumor tissue. Therefore the effect of a chemotherapeutic agent may be improved by interfering with the binding between AGP and the chemotherapeutic agent.

Hence, the invention also relates to a method for the screening of test compounds that may improve the efficacy of a chemotherapeutic agent in the chemotherapy of ovarian cancer comprising the steps of providing an assay capable of determining the binding of alpha-1 acid glycoprotein to said chemotherapeutic agent, determining the binding of alpha-1 acid glycoprotein to said chemotherapeutic agent in the presence of said test compound, comparing the level of binding of alpha-1 acid glycoprotein to said chemotherapeutic agent in the presence of said test compound with a control level of binding of alpha-1 acid glycoprotein to said chemotherapeutic agent, wherein a decreased level of binding of alpha-1 acid glycoprotein to said chemotherapeutic agent in the presence of said test compound indicates that the test compound may improve the efficacy of a chemotherapeutic agent in the therapy of ovarian cancer.

### Detailed description of the invention

The acute phase protein alpha-1 acid glycoprotein (AGP) is synthesized by the liver and its plasma levels rise in response to acute phase syndromes and several pathological conditions such as infection, burns, inflammation, following surgery, rheumatoid arthritis and cancer [4,5]. Alpha-1 acid glycoprotein has also been referred to in the literature as orosomucoid, AGP, alpha (1) acid glycoprotein and acid seromucoid.

In the last decades, AGP was investigated as a possible serum tumor marker as its preoperative levels were found to be significantly increased in patients with several types of cancer, including ovarian cancer [6,7]. Furthermore, AGP has been described as an appropriate marker for disease progression and prognosis in cancer of the esophagus, stomach, bowel, lung, liver and pancreas [6].

Although most research so far has been focused on hepatic synthesis of AGP and its release into the serum, local extra-hepatic synthesis of AGP has also been described [5]. First evidence for active synthesis of AGP outside the liver was obtained in human breast epithelial cells but it was also detected in many other tissues [16, 5]. Today, there is a growing body of evidence that the acute phase response may take place in extra-hepatic cell types, notably epithelial cells, at the site of the initial acute phase reaction [5,16,17].

In the present study, we report the presence of AGP in the cyst fluid of epithelial ovarian tumors. We studied the relationship between the AGP level in ovarian cyst fluid (oCF) and the clinical response and survival of patients with ovarian cancer, in particular in patients treated with platinum-based chemotherapy.

We obtained ovarian cyst fluid samples from 53 individual patients diagnosed with histologically proven epithelial ovarian cancer (EOC). As controls we obtained samples from 11 patients with borderline and 22 patients with benign epithelian ovarian tumors (example 1).

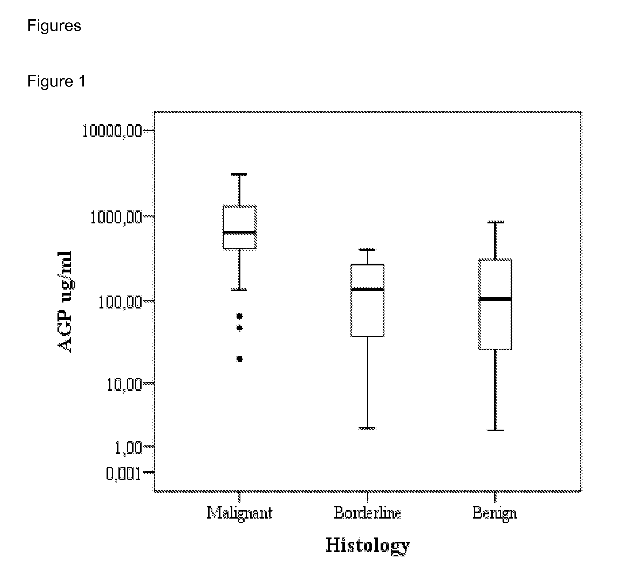

Figure 1 shows the boxplots of the AGP concentrations in ovarian cyst fluid samples from the three different tumor types. Median (interquartile range) concentrations were 639 (886), 137 (261), and 106 (285) µg/ml, for patients with malignant, borderline and benign ovarian tumors, respectively. Significantly higher concentrations of AGP were found in cyst fluid from malignant ovarian tumors compared to borderline and benign tumors (p<0.001, Kruskal Wallis test).

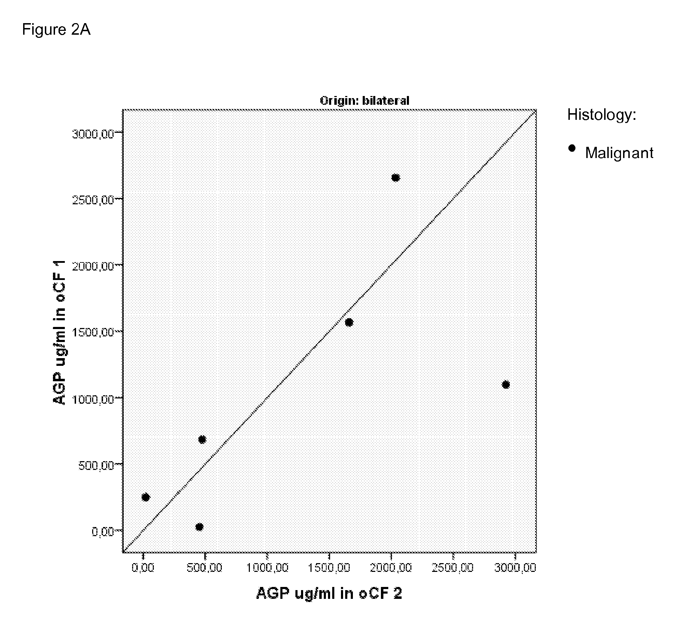

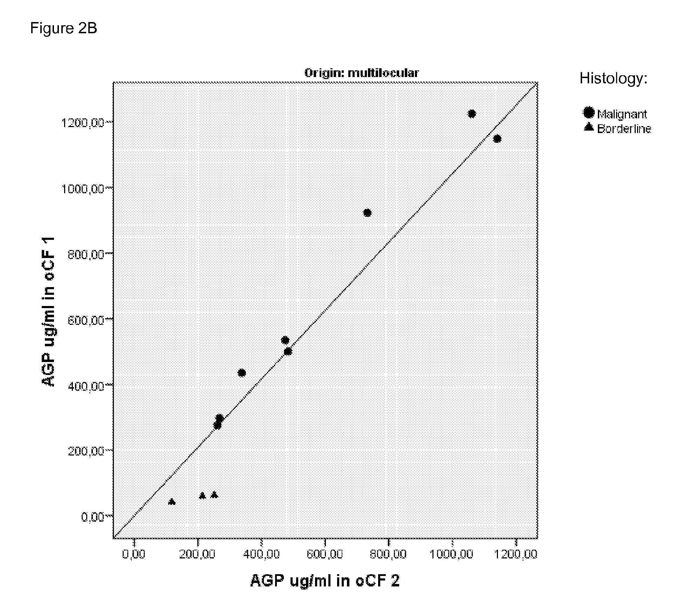

Multiple cysts, either from the same tumor or from the contra-lateral tumor in bilateral cases, were analyzed from 17 patients (malignant tumors n=14; borderline tumors n=3). The results are shown in figure 2.

The correlation between AGP levels of two cysts was high in unilateral tumors (p<0.001, R=0.974, Figure 2B, Pearson's correlation) and moderately high in bilateral tumors (R=0.669, Figure 2A, Pearson's correlation). This indicates that the cyst fluid AGP level from an ovarian cyst is remarkably consistent within one tumor, and even in most bilateral tumors.

We also determined the relation between the AGP concentration in ovarian cyst fluid and pathology. Table 1 shows the clinicopathological outcomes and median (range) concentration of AGP (µg/ml) for the 53 patients with EOC. Significantly higher AGP concentrations were found for patients with tumor grade 2 and 3 compared to patients with tumor grade 1 (p=0.029), and for patients with residual tumor after surgery of more than 1 cm compared to patients with residual tumor of 1 cm or less (p=0.002).

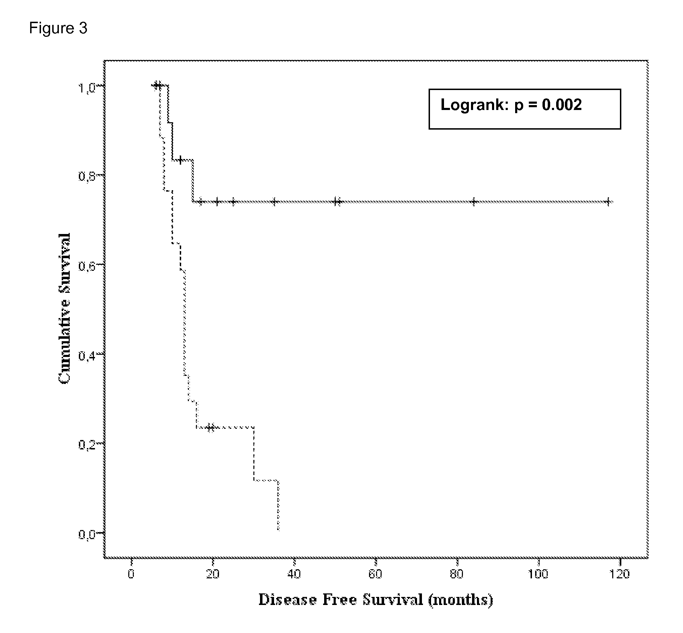

Survival analysis was performed for patients who received complete chemotherapeutic treatment after surgery (n=32). No difference in AGP level was found between patients who received platinum and paclitaxel and patients who received platinum and cyclophosphamid (p=0.195). Figure 3 shows the Kaplan-Meier curves of DFS for patients with AGP values above (n=17) and below (n=15) 500 µg/ml. Patients with high levels of AGP had a significantly shorter disease free survival (DFS) than patients with a low AGP level (logrank test: p=0.002, and p=0.006 Cox regression Table 2). After 15 months, 71% of patients with a high AGP level showed recurrence of disease compared to 36% of patients with a low AGP level.

**Table 1. Association of AGP in oCF and pathology in 53 patients with EOC.**

| | **n(%)** | **AGP (µg/ml)** | **P-** 0.887* | **Correlation#** |
|---|---|---|---|---|
| **Age** | | | | |
| < 57 years | 25 | 650 (795) | | |
| ≥ 57 years | 28 | 570 (963) | | |
| | | | | |
| **FIGO stage** | | | 0.143* | |
| ≥ IIa | 21 | 580 (802) | | |
| > IIa | 31 | 650 (884) | | |
| unknown | 1 (2) | | | |
| | | | | |
| **Tumor grade** | | | **0.029**** | 0.277 |
| 1 | 15 | 436 (495) | a | |
| 2 | 16 | 1172 (1128) | b | |
| 3 | 19 | 747 (2106) | b | |
| unknown | 3 (6) | | | |
| | | | | |
| **Histology** | | | 0.072** | |
| Serous | 23 | 747 (884) | | |
| Mucinous | 14 | 436 (435) | | |
| Endometrioid | 9 (17) | 1143 (1055) | | |
| Other | 7 (14) | 615(793) | | |
| **Residual tumor** | | | **0.002*** | 0.377 |
| ≥ 1cm | 43 | 505 (505) | | |
| ≥ 1 cm | 9 (17) | 1304 (837) | | |
| unknown | 1 (2) | | | |
| | | | | |
| **Malignant cells in** | | | 0.888* | |
| Yes | 27 | 628 (782) | | |
| No | 22 | 643 (824) | | |
| unknown | 4 (7) | | | |
| | | | | |
| **Preoperative CA 125** | | | 0.564* | |
| ≤141 U/ml | 24 | 597 (511) | | |
| >141 U/ml | 24 | 639 (882) | | |
| unknown | 5 (10) | | | |
| | | | | |
| **Total** | **53** | 639 (886) | | |

| | | | | |
|---|---|---|---|---|
| *Mann-Whitney U-test; **Kruskal-Wallis test; a and b denote categories that differ significantly; # Spearman's rank correlation (rho) | | | | |

We also did not find a relation between oCF AGP and preoperative serum CA 125 level. Furthermore, AGP did not differ between patients with mucinous tumors and patients with other histological subtypes.

In this study, AGP in the total group of EOC patients (n=53), did not show any relationship with FIGO stage, which may indicate that AGP in oCF should be considered as an independent and thus additional predictor of clinical outcome and survival. Moreover, although oCF AGP levels were significantly higher in patients with a high tumor grade and/or a suboptimal debulking, strength of the relationship was weak (R=0.277 and R=0.377, respectively). Therefore, AGP does not show a relationship with any of the clinicopathological parameters.
Table 2 shows the hazard ratio with 95% Cl, using the univariate proportional hazard model.

**Table 2: Univariate Cox regression analysis of DFS of 32 patients**

| | | **patients (n=32)** | |
|---|---|---|---|
| | | HR (95% Cl)^{#} | P |
| **Age** | | | 0.616 |
| | < 57 years | 1.00 | |
| | ≥ 57 years | 0.789 (0.31-2.00) | |
| **FIGO stage** | | | **0.002** |
| | I-II | 1.00 | |
| | III-IV | 27.21 (3.45-214.74) | |
| **Tumor grade** | | | 0.630 |
| | 1 | 1.00 | |
| | 2+3 | 1.00 (0.99-1.02) | |
| **Residual tumor** | | | **0.018** |
| | ≤ 1cm | 1.00 | |
| | > 1cm | 3.92 (1.26-12.134) | |
| **Malignant cells in** | | | **0.019** |
| | Yes | 6.06 (1.35-27.17) | |
| | No | 1.00 | |
| **Preoperative CA 125** | | | **0.041** |
| | ≤141 U/ml | 1.00 | |
| | >141 U/ml | 3.89 (1.06-14.29) | |
| **AGP in oCF** | | | **0.006** |
| | ≤500 µg/ml | 1.00 | |
| | >500 µg/ml | 5.89 (1.66-20.88) | |

| | | | |
|---|---|---|---|
| ^{#}Hazard Ratio (95% confidence interval) | | | |

Most significant predictors of DFS in patients that received chemotherapy were FIGO stage and AGP level (p<0.01). The presence of malignant cells in ascites and preoperative CA 125 value were also significant predictors of DFS (p<0.05).

In multivariate analysis with AGP level, preoperative CA 125 level, and presence of malignant cell in ascites, AGP was the only independent predictor of survival (table 2).

In conclusion, we have found that alpha-1 acid glycoprotein (AGP) concentrations were significantly higher in cyst fluid of patients with ovarian cancer (n=53) as compared to AGP concentrations in cyst fluid of patients with benign (n=22) and borderline (n=11) ovarian tumors. We also found that AGP concentrations were significantly higher in cyst fluid of patients with a poor disease outcome compared to patients with a relatively good disease outcome. In other words, patients with high levels of oCF AGP that received platinum-based chemotherapy showed a significantly shorter DFS compared to those with low levels of oCF AGP receiving chemotherapy. Moreover, AGP seemed to be an independent prognostic factor for survival in multivariate Cox regression analysis.

Accordingly, the invention provides an in vitro method for predicting the outcome of chemotherapy in ovarian cancer comprising the steps of providing a test sample comprising ovarian cyst fluid, determining the level of alpha-1 acid glycoprotein in the test sample and comparing the level of alpha-1 acid glycoprotein in the test sample with a control level of alpha-1 acid glycoprotein wherein a level of alpha-1 acid glycoprotein in the test sample higher than a control level of alpha-1 acid glycoprotein indicates that the individual is at an increased risk for recurrent disease.

Ovarian cyst fluid can be easily obtained after surgery and levels of AGP may be determined using conventional techniques, an enzyme-linked immune sorbent assay is thereby preferred. The skilled person will therefore have no difficulty in performing the method according to the invention.

Besides its role as an acute phase protein, AGP is the next important drug binding protein after albumin [4]. As human serum albumin is mainly responsible for the binding of acidic drugs, AGP binds many basic and neutral drugs [4]. Increase of AGP during pathological conditions can considerably alter the free plasma fraction of the drug without affecting its total concentration [8]. This means that for anticancer drugs with high binding affinity for AGP or a small therapeutic index, increased levels of AGP may be related to treatment failure [9]. This mechanism has been described for patients with lung cancer, where the level of AGP appeared to be an independent predictor of response to docetaxel [10, 11]. In the 1980s, a small number of studies have been performed investigating the association between serum AGP level and response to chemotherapy of patients with ovarian cancer [12-15]. Results of these studies are conflicting, and to the best of our knowledge, no research has been performed investigating the direct binding affinity of AGP with cisplatinum and cyclophosphamide, which was the standard chemotherapy regimen for ovarian cancer at that time.

We found that high levels of AGP in cyst fluid of these ovarian cancer patients lead to an increased metabolism of chemotherapeutics and thus to a diminished effect of the chemotherapy on the tumor tissue. Therefore the effect of a chemotherapeutic agent may be improved by interfering with the binding between AGP and the chemotherapeutic agent.

Hence, the invention also relates to a method for the screening of test compounds that may improve the efficacy of a chemotherapeutic agent in the chemotherapy of ovarian cancer comprising the steps of providing an assay capable of determining the binding of alpha-1 acid glycoprotein to said chemotherapeutic agent, determining the binding of alpha-1 acid glycoprotein to said chemotherapeutic agent in the presence of said test compound, comparing the level of binding of alpha-1 acid glycoprotein to said chemotherapeutic agent in the presence of said test compound with a control level of binding of alpha-1 acid glycoprotein to said chemotherapeutic agent, wherein a decreased level of binding of alpha-1 acid glycoprotein to said chemotherapeutic agent in the presence of said test compound indicates that the test compound may improve the efficacy of a chemotherapeutic agent in the therapy of ovarian cancer.

### Legend to the figures

Figure 1. Boxplots of AGP concentrations in cyst fluid of patients with malignant (n=53), borderline (n=11) and benign (n=22) ovarian tumors.
Figure 2. Double cyst fluid samples (n=17). (A) represents AGP values of double samples from both ovaries (n=6). (B) represents AGP values of double samples of separate cysts from one ovary (n=11).
Figure 3. Kaplan-Meier disease free survival curve of patients with epithelial ovarian cancer that received adjuvant chemotherapy (n=32). The dotted line represent DFS of patients with high (>500 µg/ml; n=17) oCF AGP and the black line represents DFS of patients with low (≤500 µg/ml; n=15) oCF AGP.

### Examples

### Example 1: Patients

This study included 53 patients diagnosed with histologically proven epithelial ovarian cancer (EOC) of whom ovarian cyst fluid (oCF) was collected and stored after primary surgery. This multicentre study was performed at the Radboud University Nijmegen Medical Centre (RUNMC) (n=20) and at 9 regional hospitals in The Netherlands (n=33) in the period between January 1996 and January 2008. Surgery was always performed by a (travelling) gynaecologist specialized in oncology from the RUNMC. As controls we used oCF samples from 11 patients with borderline and 22 patients with benign epithelial ovarian tumors, which were obtained after primary surgery at the RUNMC. Survival analysis was performed for the group of patients with EOC that received chemotherapy (n=32). Informed consent was obtained from all participants.

Age: Median age at diagnosis for patients with EOC was 57 years (n=53; range: 32-89), 56 years (n=11; range: 43-82) for patients with borderline tumors, and 52 years (n=22; range: 20-70) for patients with benign tumors. Age did not differ between patients groups (p=0.053, Mann-Whitney U-test).

FIGO stage: Of the 21 patients with early FIGO stage EOC, 11 had la, 1 had Ib, and 9 patients had FIGO stage Ic. Of the patients 31 with advanced FIGO stage EOC, 2 had IIb, 2 had IIc, 2 had at least stage III, 2 had IIIa, 7 had IIIb, 11 had IIlc and 5 patients had FIGO stage IV. For 1 patient, FIGO stage could not be obtained.

The remaining clinicopathological data of the patients with EOC are listed in Table 1.

### Example 2: Cyst fluid collection

oCF samples (n=103) were collected immediately after surgical removal of the tumor. Of 17 patients (n=14 with malignant tumors; n=3 with borderline tumors), two samples were obtained from either both ovaries (n=6) or from two separate cysts of one ovary (n=11). After cooled transport to the laboratory, the samples were centrifuged at 3000 x g for ten minutes and the supernatant was stored at -35°C in small portions until use.

### Example 3: AGP measurements

AGP concentrations were measured using an ELISA essentially as reported previously [18]. In summary, ELISA plates were coated overnight with polyclonal anti-human AGP obtained from Dako (Glostrup, Denmark). Diluted plasma samples and a standard dilution series with human and rAGP, were added to the plate. Detection was carried out with a biotinylated polyclonal rabbit anti-human AGP IgG, followed by peroxidase-conjugated streptavidin and substrate essentially as described by De Vries et al [19]. Determination of the levels of AGP was carried out without knowledge of the histological or clinical outcome.

### Example 4: Clinicopathologic characteristics

Complete pathological reports of all patients were reviewed for correct histopathological diagnosis (primary EOC, histological tumor subtype and grade) by one pathologist from the RUNMC, specialized in gynaecological oncology. From the medical records of the patients diagnosed with EOC, the following clinicopathologic characteristics were retrospectively retrieved: age at diagnosis, FIGO stage, residual tumor after surgery, preoperative CA 125 level (U/ml), presence of malignant cells in ascites, chemotherapeutic treatment, date of tumor recurrence and date of death. For some patients, information on the following parameters was missing:
FIGO stage (n=1), tumor grade (n=3), histology (n=7), residual tumor after surgery (n=1), presence of malignant cells in ascites (n=4), preoperative CA 125 level (n=5) (Table 1). Staging was performed according to the criteria of the International Federation of Gynaecologists and Obstetricians (FIGO) [20]. Histopathological tumor type and grade were classified according to the World Health Organization (WHO) criteria [21]. Chemotherapeutic treatment was defined as complete if in the first line combination chemotherapy for 6 courses was given, always including a platinum-based agent, and if start of treatment was within three weeks after surgery. Additional information regarding recurrence of disease was collected for patients who received platinum-based chemotherapy. Recurrence of disease was defined as a measurable lesion detected by computed tomography, magnetic resonance imaging and/or ultrasonography. Follow-up period should be at least 6 months after the last course of chemotherapy for all patients to be included in our series.

### Example 5: Statistical analyses

For patients of whom two AGP values were obtained from different cysts, the mean AGP level was taken for statistical analysis. Variables regarding patient characteristics were grouped in the following manner: FIGO stage: Ia-IIa vs. IIb-IV; tumor grade: 1 vs. 2 vs. 3; histology: serous vs. mucinous vs. endometrioid; residual disease: less than 1 cm (definition of optimal cytoreductive surgery) vs. equal or greater than 1 cm (definition of suboptimal debulking); ascites: presence of malignant cells vs. no malignant cells; preoperative CA 125: equal or less than 141 U/ml (≤median value) vs. greater than 141 U/ml (>median value).

Differences in AGP concentration between groups of patients were tested for statistical significance using the Mann-Whitney U-test in case of two groups and the Kruskal Wallis test in case of more than two groups.

Survival techniques were used to study the time to recurrence and time to death. The disease free survival (DFS) was defined as the time interval from the date of the last course of chemotherapy to the date of recurrence, death or last follow-up. The AGP oCF value of 500 µg/ml was used for dividing patients into two groups after a statistically significant difference in DFS was found with logistic regression analysis. The Kaplan-Meyer estimates were calculated of the patients with AGP oCF values below 500 µg/ml and above 500 µg/ml, respectively. Subsequently, the log-rank test was used to test their difference for statistical significance. An univariate proportional hazards model was used to study the influence of the clinicopathological parameters on DFS separately. FIGO stage, histology and residual tumor after surgery were not studied because of the limited number of patients within the different subgroups. The hazard ratios with the corresponding 95% confidence interval are presented. A multivariate proportional hazards model with selection procedures was used to find the clinicopathological parameters that independently contribute to a decreased time to recurrence. The adjusted hazard ratio's (HR) with the corresponding 95% confidence interval (Cl) of the final model are presented.

P-values less than <0.05 were considered statistical significant. All statistical analyses were performed using the software package SPSS 14.0 for Microsoft Windows (SPSS Inc., Chicago, IL, USA).

### Example 6: Histopathology

Of patients with EOC, 23 had serous tumors, 14 had mucinous tumors, 9 had endometrioid tumors, 2 had clear cell tumors, 1 had a mixed cell tumor, 1 had an undifferentiated adenocarcinoma and 3 had adenocarcinomas not otherwise specified. Of patients with borderline tumors, mucinous and serous tumors were found in 8 and 3 patients, respectively. Histological subtypes of patients with benign tumors included 7 serous, 11 mucinous, 1 mixed type tumor, and 3 simple epithelial cysts.

### Example 7: Chemotherapy

Of the total number of 53 patients with EOC, 32 patients received complete first line chemotherapeutic treatment with 6 courses of platinum-based chemotherapy, 4 patients received neo-adjuvant chemotherapy and 17 patients did not receive chemotherapy at all. Of the 32 patients who received adjuvant chemotherapy, 20 received a combination of platinum and paclitaxel and 12 received a combination of platinum and cyclophosphamid.

### Example 8: Survival

Median follow-up period was 41 months (range: 9-254 months). DFS in the chemotherapy group (n=32) ranged from 6 to 117 months (median 13 months).Within the follow-up period 18 patients (56%) showed recurrence of disease and 10 patients (31%) died.

### References

[1] Jemal A, et al., CA Cancer J Clin 2007;57:43-66.
[2] Armstrong DK. Oncologist 2002;7 Suppl 5:20-8.
[3] Gonzalez-Martin A.. Int J Gynecol Cancer 2005;15 Suppl 3:241-6.
[4] Israili ZH, and Dayton PG. Drug Metab Rev 2001;33:161-235.
[5] Fournier T,et al., Biochim Biophys Acta 2000;1482:157-71.
[6] Hashimoto S, et al., Cancer 2004;101:2825-36.
[7] Fish RG, et al., Eur J Cancer Clin Oncol 1984;20:625-30.
[8] Kremer JM, et al., Pharmacol Rev 1988;40:1-47.
[9] Jackson PR, et al., Clin Pharmacol Ther 1982;32:295-302.
[10] Bruno R, et al., Clin Cancer Res 2003;9:1077-82.
[11] Yildirim A, et al., Med Sci Monit 2007;13:CR195-CR200.
[12] Piver MS, et al., Gynecol Oncol 1988;29:305-8.
[13] Fish RG, et al., Clin Biochem 1984;17:39-41.
[14] Meerwaldt JH, et al., Gynecol Oncol 1983;16:209-18.
[15] Lukomska B, et al., Gynecol Oncol 1981;11:288-98.
[16] Gendler SJ, et al., Cancer Res 1982;42:4567-73.
[17] Dube JY, et al., Prostate 1989;15:251-8.
[18] van Dielen FM, et al., Int J Obes Relat Metab Disord 2001;25:1759-66.
[19] de Vries, B, et al., Transplantation 2004;78:1116-24.
[20] Pecorelli S, et al., Int J Gynaecol Obstet 1999;65:243-9.
[21] Servov SF, Scully RE, Sobin LH. International histologic classification of tumors. No. 9: Histologic typing of ovarian tumors. Geneva: World Health Organization, 1973
[22] Tingulstad S, et al., Obstet Gynecol 2003;101:885-91.
[23] Gadducci A, et al., Crit Rev Oncol Hematol 2008.
[24] Hogdall EV, et al., Gynecol Oncol 2007;104:508-15.

## Claims

1. An in vitro method for predicting the outcome of chemotherapy in ovarian cancer comprising the steps of:
a. providing a test sample comprising ovarian cyst fluid,
b. determining the level of alpha-1 acid glycoprotein in the test sample and
c. comparing the level of alpha-1 acid glycoprotein in the test sample with a control level of alpha-1 acid glycoprotein
wherein a level of alpha-1 acid glycoprotein in the test sample higher than a control level of alpha-1 acid glycoprotein indicates that the individual is at an increased risk for recurrent disease

2. Method for the screening of test compounds that may improve the efficacy of a chemotherapeutic agent in the chemotherapy of ovarian cancer comprising the steps of:
a. providing an assay capable of determining the binding of alpha-1 acid glycoprotein to said chemotherapeutic agent,
b. determining the binding of alpha-1 acid glycoprotein to said chemotherapeutic agent in the presence of said test compound,
c. comparing the level of binding of alpha-1 acid glycoprotein to said chemotherapeutic agent in the presence of said test compound with a control level of binding of alpha-1 acid glycoprotein to said chemotherapeutic agent,
wherein a decreased level of binding of alpha-1 acid glycoprotein to said chemotherapeutic agent in the presence of the test compound indicates that the test compound may improve the efficacy of a chemotherapeutic agent in the therapy of ovarian cancer.

3. Method according to claims 1 or 2 wherein the chemotherapy comprises a treatment with platinum based chemotherapeutics.

4. Method according to claim 1 wherein the level of alpha-1 acid glycoprotein is determined in an enzyme-linked immune sorbent assay
